Europäisches Patentamt

European Patent Office

Office européen des brevets

(19)

(11) Veröffentlichungsnummer : **0 401 243 B1**

(12) **EUROPÄISCHE PATENTSCHRIFT**

(45) Veröffentlichungstag der Patentschrift :
**02.09.92 Patentblatt 92/36**

(21) Anmeldenummer : **89902249.5**

(22) Anmeldetag : **06.02.89**

(86) Internationale Anmeldenummer :
**PCT/EP89/00107**

(87) Internationale Veröffentlichungsnummer :
**WO 89/07453 24.08.89 Gazette 89/20**

(51) Int. Cl.$^5$ : **A61K 45/06, A61K 31/66,**
**A61K 31/675, A61K 31/195,**
**A61K 31/135, A61K 31/00**

(54) **ARZNEIMITTEL MIT EINER KOMBINATION VON CYTOSTATIKA BZW. HORMONTHERAPEUTIKA UND PHOSPHONODERIVATEN.**

(30) Priorität : **15.02.88 DE 3804686**

(43) Veröffentlichungstag der Anmeldung :
**12.12.90 Patentblatt 90/50**

(45) Bekanntmachung des Hinweises auf die Patenterteilung :
**02.09.92 Patentblatt 92/36**

(84) Benannte Vertragsstaaten :
**AT BE CH DE FR GB IT LI LU NL SE**

(56) Entgegenhaltungen :
**BE-A-89 045 3**
**DE-A- 3 500 670**
**GB-A- 1 453 667**
**US-A- 4 608 368**
**Chemical Abstracts, Volume 107, Nr. 1,6, 6. July 1987 (Columbus, Ohio, US) Garattini Silvio et al: "Effect of etidronatedisodium on the interactions between malignancy and bone" see page 52, abstract 635p, & Am.J. Med. 1987 82(2A), 29-33**

(73) Patentinhaber : **Henkel Kommanditgesellschaft auf Aktien Postfach 1100 Henkelstrasse 67 W-4000 Düsseldorf-Holthausen (DE)**
Patentinhaber : **Deutsches Krebsforschungszentrum Im Neuenheimer Feld 280 W-6900 Heidelberg 1 (DE)**

(72) Erfinder : **BLUM, Helmut Bertha-von-Suttner-Strasse 30 W-4000 Düsseldorf 13 (DE)**
Erfinder : **KLENNER, Thomas Carl-Diem-Weg 6 W-6945 Hirschberg (DE)**
Erfinder : **SCHMÄHL, Dietrich Sandwingert 57 W-6900 Heidelberg (DE)**
Erfinder : **WINGEN, Franz Gartenstrasse 4 W-5204 Lohmar (DE)**

EP 0 401 243 B1

Jouve, 18, rue Saint-Denis, 75001 PARIS

## Beschreibung

Die Erfindung betrifft Arzneimittel, die durch eine Kombination von Cytostatika bzw. Hormontherapeutika und einem Phosphonoderivat gekennzeichnet sind. Die Erfindung betrifft weiterhin Verfahren zur Herstellung der genannten Arzneimittel sowie die Verwendung der Cytostatika bzw. Hormontherapeutika und der Phosphonoderivate.

Die Ergebnisse der Chemotherapie des Krebses sind nicht befriedigend. So werden z.B. bei Mammakarzinomen unabhängig vom eingesetzten Chemotherapie-Schema nur maximal in 40 % der Fälle Remissionen erreicht. Weiterhin ist die Chemotheradie von starken Nebenwirkungen begleitet. Neben akuten Nebenwirkungen wurden bei Langzeitbeobachtungen auch therapieinduzierte Zweitmalignome gefunden, die mit der Verwendung von mutagenen und karzinogenen Cytostatika in Zusammenhang gebracht werden können.

Krebsmetastasen zeigen je nach Lokalisation ein unterschiedliches Ansprechverhalten auf Chemotherapie und Hormontherapie. So haben Knochenmetastasen des Mammakarzinoms nur eine halb so gute Ansprechrate wie Weichteilmetastasen. Singuläre Knochenmetastasen werden radiotherapeutisch behandelt. Bei disseminierten Knochenmetastasen ist diese Behandlungsform meist jedoch nicht durchführbar. Hier kann Calcitonin eingesetzt werden. Calcitonin hat jedoch einen, in erster Linie analgetischen Effekt und zeigt nur eine geringe Wirkung beim Wiederaufbau des Knochens. Knochenmetastasierende Tumore (Mamma, Prostata, Lunge, Schilddrüse) stellen noch ein großes therapeutisches Problem-dar. Obwohl die Hormontherapie, z.B. mit Tamoxifen, mit einer geringen Nebenwirkungsrate behaftet ist, sind ihre Erfolge nicht befriedigend, vgl. E. Petru und D. Schmähl, Dtsch. med. Wschr. (1987), 112, 270.

Die Erfindung ist auf ein Therapeutikum mit mehreren Angriffspunkten bei knochenmetastasierenden Malignomen gerichtet. Dabei sollen Tumorzellen direkt durch Cytostatika bzw. Hormontherapeutika beeinflußt werden, während bestimmte Knochenzellen (Osteoklasten), die an der Ausbildung von Knochenmetastasen wesentlich beteiligt sind, gehemmt werden, vgl. F. Wingen et al., J. Cancer Research Clinical Oncology (1986), 111, 35.

Es wurde nun überraschend gefunden, daß dies erreicht wird, wenn man ein bestimmtes Phosphonoderivat mit einem Cytostatikum kombiniert Die Arzneimittel der Erfindung hemmen die Tumorzellen und die knochenresorbierenden Zellen. Durch Kombination mit einem Phosphonoderivat läßt sich die Cytostatikadosis deutlich reduzieren.

Phosphonate werden vorwiegend bei der Therapie von Morbus Paget, Tumorhypercalcämie und Knochenmetastasen eingesetzt

Bevorzugte Arzneimittel der Erfindung sind dadurch gekennzeichnet, daß das Phosphonoderivate 3-Amino-1-hydroxypropandiphosphonsäure ist.

Weiterhin bevorzugte Arzneimittel der Erfindung sind dadurch gekennzeichnet, daß das Phosphonoderivat in Form seiner Neutralsalze, insbesondere seiner Alkali- und Erdalkalisalze, vorliegt.

Weiterhin bevorzugte Arzneimittel der Erfindung sind dadurch gekennzeichnet, daß sie als Cytostatika Melphalan, Chlorambucil, Dacarbacin, Methotrexat, Cyclophosphamid, Vincristin, Trofosfamid, Ifosfamid, 5-Fluorouracil, Lomustine, Hydroxyharnstoff, Cisplatin, Carboplatin, Hexamethylenmelamin, Daunorubicin, Vinblastin und/oder Zorubicin, enthalten.

Weiterhin bevorzugte Arzneimittel der Erfindung sind dadurch gekennzeichnet, daß sie als Hormontherapeutika Tamoxifen, Droloxifen und/oder Methroxyprogesteronacetat enthalten.

Weiterhin bevorzugte Arzneimittel der Erfindung sind dadurch gekennzeichnet, daß sie einen Gehalt an Melphalan und 3-Amino-1-hydroxypropandiphosphonsäure, vorzugsweise in Form ihres Dinatriumsalzes derselben, aufweisen.

Die Erfindung betrifft weiterhin ein Verfahren zur Herstellung eines Arzneimittels, das dadurch gekennzeichnet ist, daß man die obengenannten Cytostatika bzw. Hormontherapeutika und das Phosphonoderivat mischt.

Die Erfindung betrifft weiterhin die Verwendung einer Kombination von Cytostatika bzw. Hormontherapeutika und Phosphonoderivaten der obengenannten Art zur Herstellung eines Arzneimittels.

Die Arzneimittel der Erfindung eignen sich insbesondere für die therapeutische Behandlung von Krebserkrankungen, insbesondere knochenmetastasierenden Malignomen. Die Verarbeitung der Wirkstoffkombinationen erfolgt nach üblichen Verfahren mit üblichen Hilfs- und Füllstoffen zu Arzneimittelformulierungen wie Kapseln, Tabletten, Lösungen, Injektionslösungen, Infusionslösungen und dergleichen.

Die Wirkung der Arzneimittel der Erfindung wurde wie folgt nachgewiesen:

Die hemmende Wirkung von Diphosphonaten auf Osteoklasten wurde bereits sowohl am Tiermodell als auch am Menschen nachgewiesen, vgl. B. Krempien et. al., Oncologie Ms, 768, 1. An in der experimentellen Chemotherapie üblichen Tiermodellen wurde jetzt nachgewiesen, daß eine Kombination eines Diphosphonats wie 3-Amino-1-hydroxypropan-diphosphonsäure (APD) mit einem Cytostatikum (Melphalan, MEL) eine synergistische Wirksamkeit bezüglich des Tumorvolumens und bezüglich der Tumorremissionen zeigt. Die Wirksamkeit der Kombinationstherapie war weitaus besser als die Wirksamkeit der einzelnen Komponenten (APD oder MEL) an diesem Modell (vgl. Tabelle 1). Es konnte insbesondere gezeigt werden, daß sich durch zusätzliche Gabe eines Diphosphonats zu einem Cytostatikum die Dosis des zu verabreichenden Cytostatikums deutlich reduzieren läßt.

## Tabelle 1

| Substanz (Totaldosis) | Tumorvolumen nach 7 Wochen (Ende der Therapie | % Tumorremissionen (max. innerhalb Therapie) |
|---|---|---|
| Unbehandelt | 43,2 | 3 |
| APD (353 mg) | 5,2 | 14 |
| APD (176 mg) | 30,7 | 12 |
| MEL (18 mg) | 11,4 | 4 |
| MEL (9 mg) | 18,2 | 3 |
| APD + MEL (353 mg + 18 mg) | 0,2 | 47 |
| APD + MEL (353 mg + 9 mg) | 2,0 | 23 |

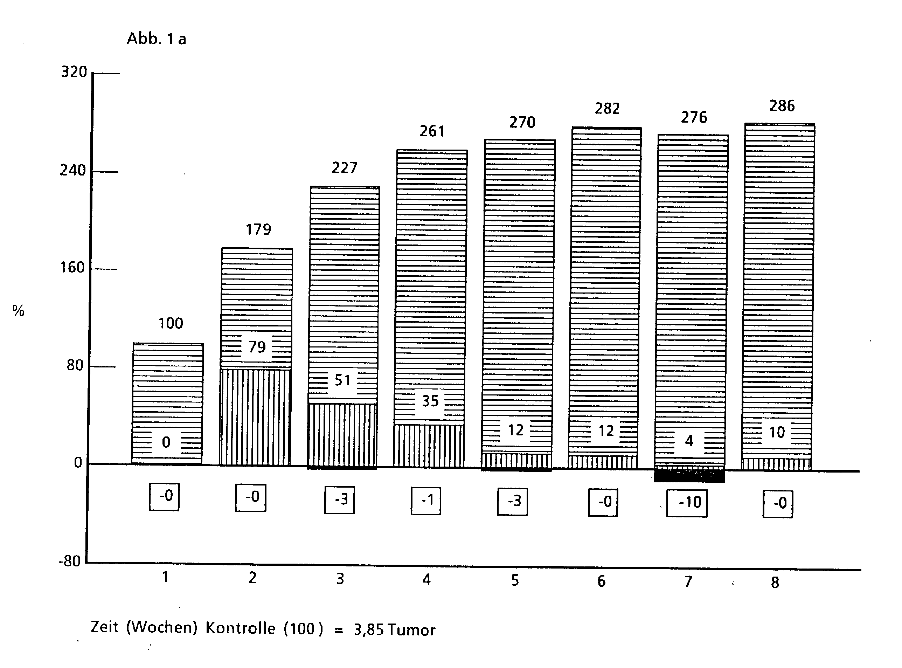

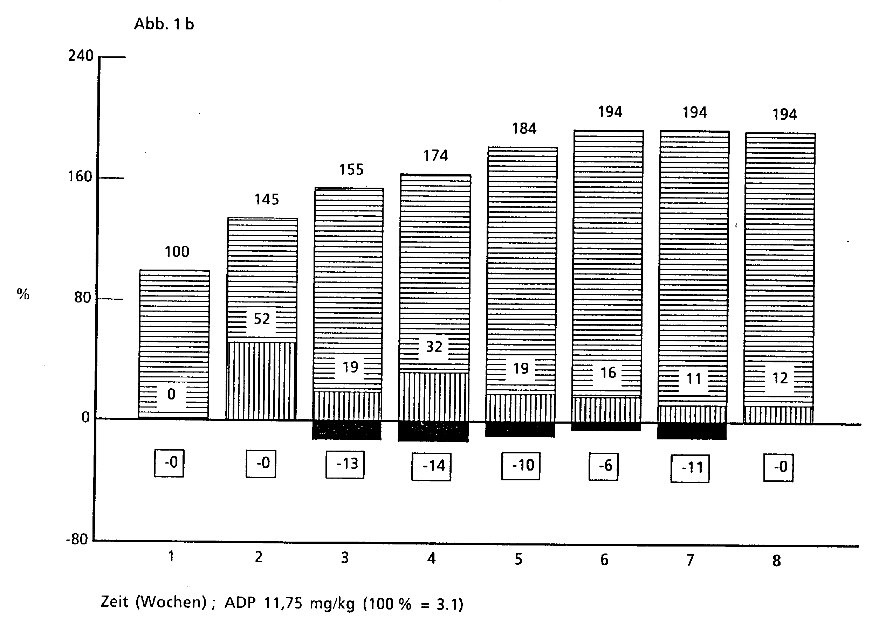

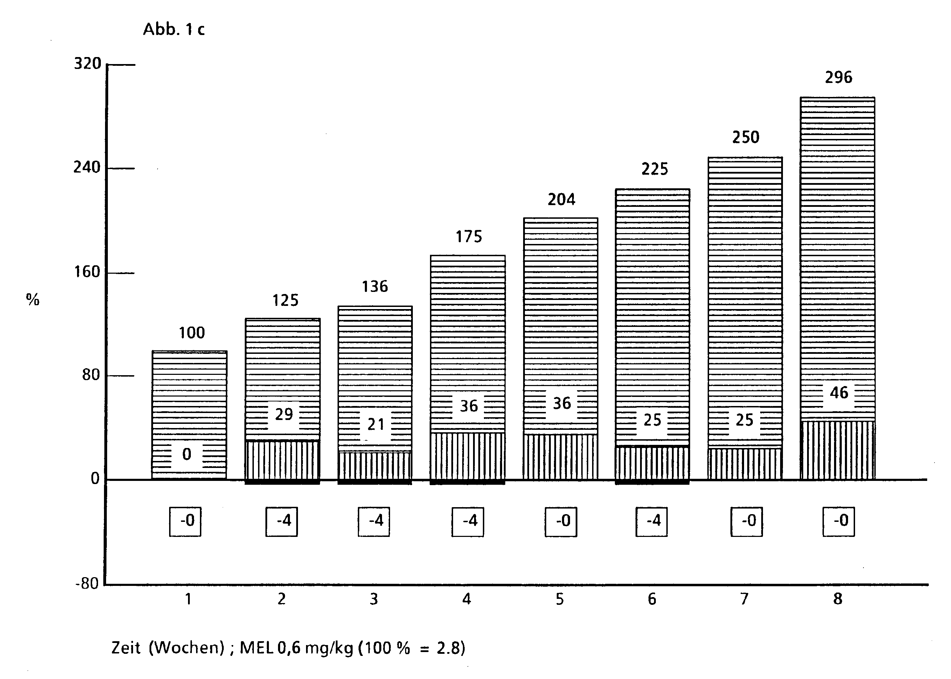

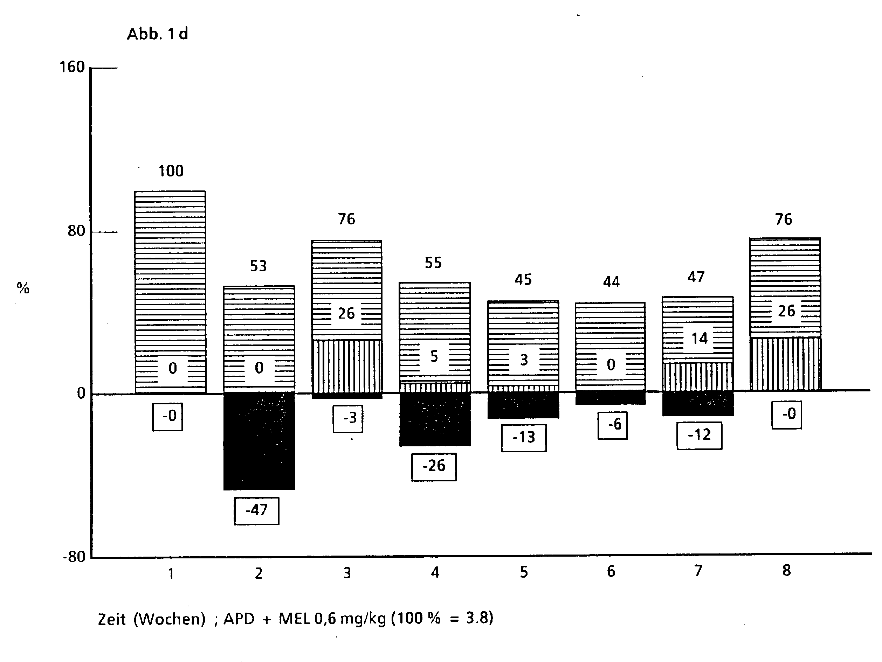

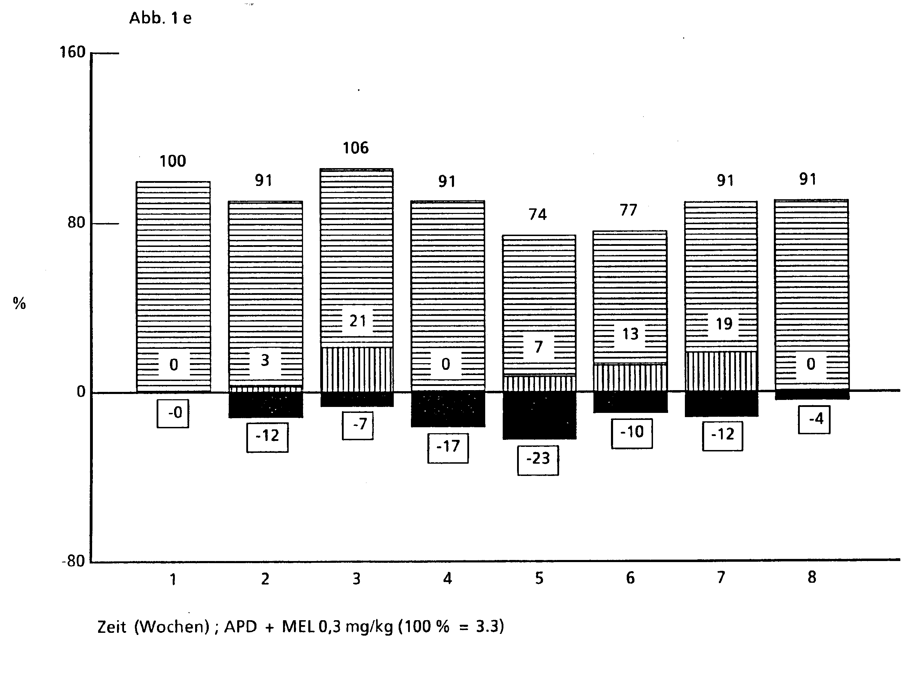

Die erhaltenen Ergebnisse sind in den Abbildungen 1 a bis e und 2 a bis c grafisch dargestellt. Bei den Abbildungen 1 a bis e geben die Säulen die Gesamtzahl an Tumoren, die vollständigen Remissionen und die Tumorbildung der Kontrollgruppe sowie der mit APD, MEL und mit 2 Kombinationen von APD + MEL behandelten Gruppen wieder. Die ursprüngliche Anzahl an Tumoren wurde als 100 % gewählt. Alle Differenzen in den folgenden Wochen wurden auf diesen Ausgangswert bezogen. Die Grobschraffierung bedeutet neu aufgetretene Tumore; schwarz bedeutet Tumorremissionen. Man beachte die verschiedenen Maße der Y-Achse.

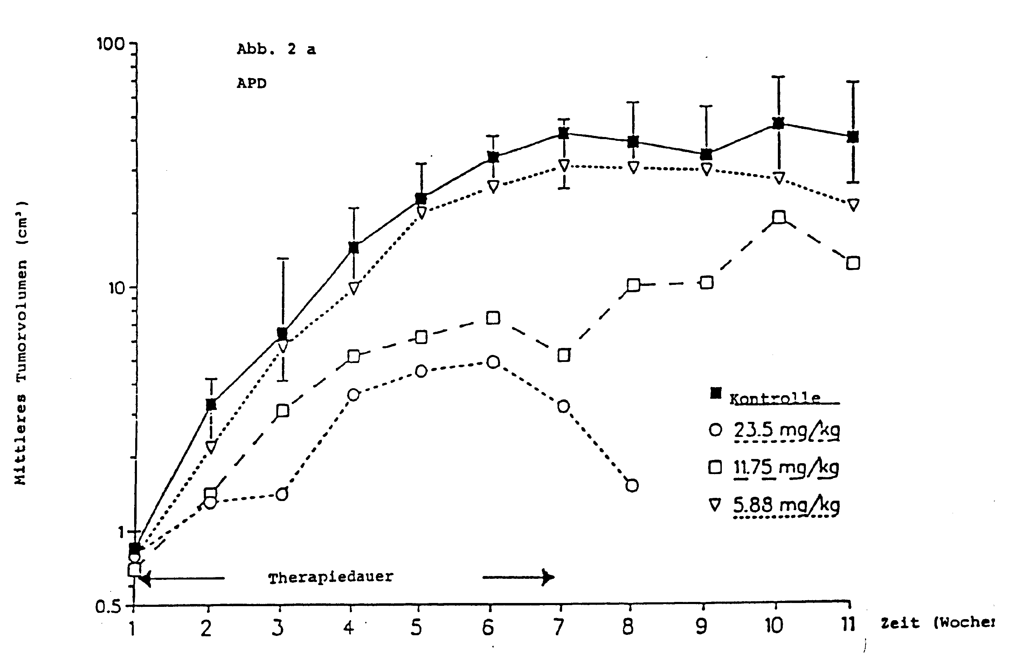

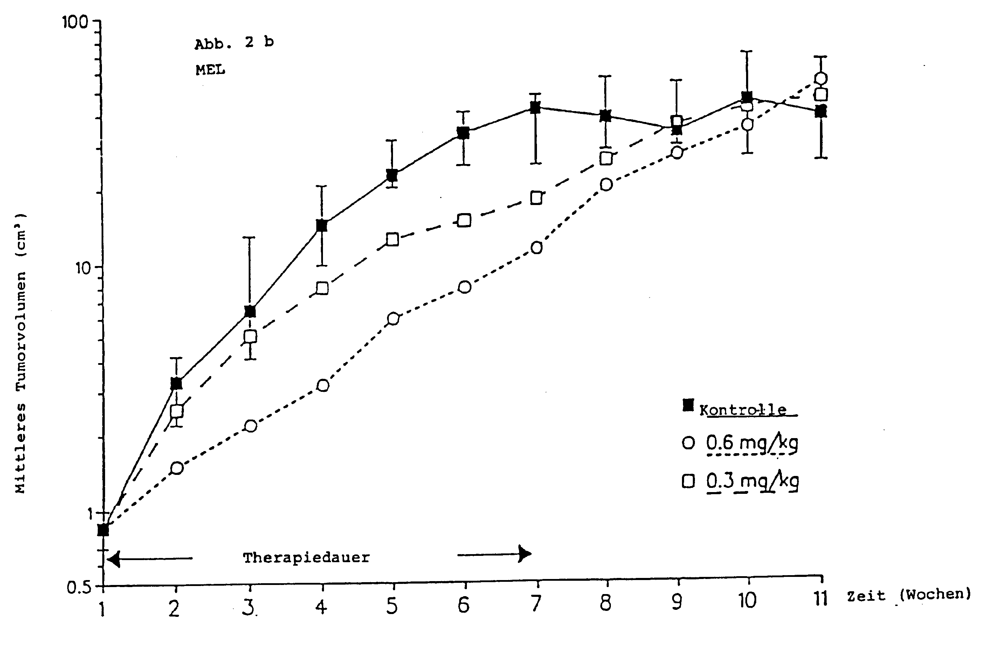

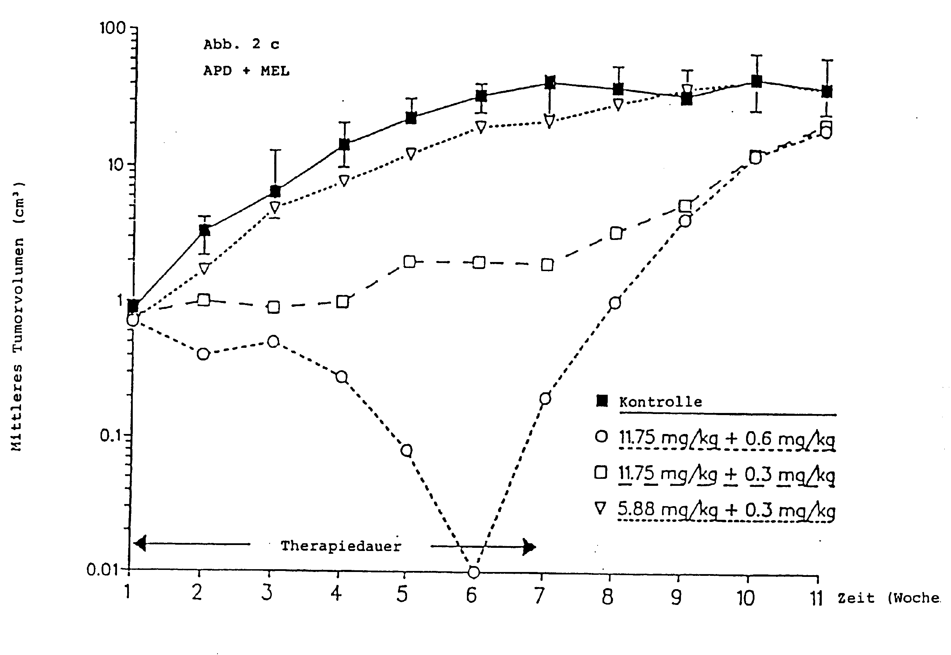

Bei den Abbildungen 2 a bis c ist das im logarithmischen Maßstab wiedergegebenen Verhältnis zwischen dem mittleren Tumorvolumen (cm$^3$) und der Zeit (Wochen) dargestellt; Ergebnisse der Therapie mit APD, MEL und der Kombination von APD + MEL sind ebenfalls dargestellt; die Änderung der Skala der Y-Achse bei 2 c ist zu beachten. Wie sich aus den Abbildungen 2 a bis c ergibt, zeigt sich beim Vergleich der Tumorvolumenentwicklung nach Therapie mit 0,3 mg APD pro kg und der Kombinationstherapie mit zusätzlich 11,75 mg APD/kg ein deutlich besserer Effekt für die Kombination.

Im folgenden werden bevorzugte Rezepturbeispiele für die Arzneimittel der Erfindung angegeben.

Beispiel 1.

Herstellung einer Tablette:

| | |
|---|---|
| Melphalan | 5 g |
| Diphosphonat (Na$_2$APD) | 100g |
| Tablettose | 200 g |
| Avicel | 30 g |
| Aerosil | 5 g |
| Mg-Stearat | 5 g |
| Primojel | 5 g |

Die Inhaltsstoffe werden gemischt und auf einer geeigneten Tablettenpresse direkt verpreßt zu Tabletten mit einem Gewicht von 450 mg.

Beispiel 2.

Herstellung einer Tablette:

| | |
|---|---|
| Tamoxifendihydrogencitrat | 15,2 g |
| Diphosphonat (Na$_2$APD) | 100 g |
| Tablettose | 300 g |
| Avicel | 30 g |
| MG-Stearat | 5 g |
| Primojel | 5 g |

Die Inhaltsstoffe werden gemischt und auf einer geeigneten Tablettenpresse direkt verpreßt zu Tabletten mit einem Gewicht von 460 mg.

Beispiel 3.

Herstellung einer Infusionslösung:

a) Diphosphonat-Lösung
700 g Diphosphonat (Na$_2$APD) werden in 250 l isotonischer Kochsalzlösung unter Rühren gelöst. Die Lösung wird steril filtriert und in 250 mg Infusionsbehälter abgefüllt. Die gesamte Herstellung erfolgt unter aseptischen Bedingungen.
b) Melphalan-Lösung
50 g Melphalan werden 1 l tert.-Butanol gelöst. Die Lösung wird in 5 ml Vials abgefüllt und gefriergetrocknet. Der gesamte Herstellungsprozeß läuft unter aseptischen Bedingungen.
– Lösungsampulle:
2 %-ige Chlorwasserstofflösung wird mit Ethanol im Verhältnis 9 : 1 gemischt und nach Sterilfiltration in 2 ml-Ampullen abgefüllt.
– Verdünnungsampulle:
5 l Propylenglykol werden mit 4 l Phosphatpuffer-Lösung gemischt und nach Sterilfiltration in 10 ml-Ampullen abgefüllt. Vor der Anwendung wird das Lyophilisat mit der Lösungsampulle in Lösung gebracht und anschließend mit der Verdünnungsampulle verdünnt. Diese Lösung wird in 250 ml isotonische Kochsalzlösung gegeben.
c) Die Lösungen a) und b) werden dann gleichzeitig über separate Infusionssysteme infundiert.

**Patentansprüche**

1. Arzneimittel, enthaltend eine Kombination von Cytostatika bzw. Therapeutika für die Hormontherapie von Tumoren und einem Phosphonoderivat, dadurch gekennzeichnet, daß es sich bei dem Phosphonoderivat um 3-Amino-1-hydroxypropandiphosphonsäure (APD) handelt und daß die Cytostatika bzw. die Therapeutika für die Hormontherapie von Tumoren und 3-Amino-1-hydroxypropandiphosphonsäure in molaren Verhältnissen von 25 : 1 bis 1 : 20 vor- handen sind und die Phosphonoverbindung sowie gegebenenfalls die Cytostatika bzw. Hormontherapeutika in Form ihrer pharmazeutisch verträglichen Salze vorliegen, gegebenenfalls zusammen mit üblichen pharmazeutischen Trägern und/oder Verdünnungsmitteln.

2. Arzneimittel nach Anspruch 1, dadurch gekennzeichnet, daß die 3-Amino-1-hydroxypropandiphosphonsaure in Form ihrer Neutralsalze, insbesondere ihrer Alkali- und Erdalkalisalze, vorliegt.

3. Arzneimittel nach Anspruch 1 oder 2, dadurch gekennzeichnet, daß sie als Cytostatika Melphalan, Chlorambucil, Dacarbacin, Methotrexat, Cyclophosphamid, Vincristin, Trophosphamid, Iphosphamid, 5-Fluorouracil, Lomustine, Hydroxyharnstoff, Cisplatin, Carboplatin, Hexamethylenmelamin, Daunorubicin, Vinblastin und/oder Zorubicin enthalten.

4. Arzneimittel nach einem der Ansprüche 1 bis 3, dadurch gekennzeichnet, daß sie als Therapeutika für die Hormontherapie von Tumoren Tamoxifen, Droloxifen und/oder Methroxyprogesteronacetat enthalten.

5. Arzneimittel nach einem der Ansprüche 1 bis 4, dadurch gekennzeichnet, daß sie einen Gehalt an Melphalan und 3-Amino-1-hydroxypropandiphosphonsäure vorzugsweise in Form ihres Dinatriumsalzes aufweisen.

**6.** Verfahren zur Herstellung eines Arzneimittels nach einem der Ansprüche 1 bis 5, dadurch gekennzeichnet, daß man die Cytostatika bzw. Therapeutika für die Hormontherapie von Tumoren und 3-Amino-1-hydroxypropandiphosphonsäure mischt.

**7.** Verwendung einer Kombination von Cytostatika bzw. Hormontherapeutika und Phosphonoderivaten gemäß einem der Ansprüche 1 bis 6 zur Herstellung eines Arineimittels für die Chemotherapie von Tumoren.

## Claims

**1.** Medicaments containing a combination of cytostatic agents or therapeutic agents for the hormone therapy of tumours and a phosphono derivative, characterized in that the phosphono derivative is 3-amino-1-hydroxypropane diphosphonic acid (APD) and in that the cytostatic agents or therapeutic agents for the hormone therapy of tumours and the 3-amino-1-hydroxypropane diphosphonic acid are present in molar ratios of 25:1 to 1:20 and the phosphono compound and optionally the cytostatic agents or hormone therapeutic agents are present in the form of their pharmaceutically acceptable salts, optionally together with typical pharmaceutical excipients and/or diluents.

**2.** Medicaments as claimed in claim 1, characterized in that the 3-amino-1-hydroxypropane diphosphonic acid is present in the form of its neutral salts, more particularly its alkali metal or alkaline earth metal salts.

**3.** Medicaments as claimed in claim 1 or 2, characterized in that they contain melphelan, chlorambucil, dacarbazine, methotrexate, cyclophosphamide, vincristine, trophosphamide, ifosfamide, 5-fluorouracil, lomustine, hydroxyurea, cisplatin, carboplatin, hexamethylene melamine, daunorubicin, vinblastine and/or zorubicin as cytostatic agents.

**4.** Medicaments as claimed in any of claims 1 to 3, characterized in that they contain tamoxifen, droloxifen and/or methroxyprogesterone acetate as therapeutic agents for the hormone therapy of tumours.

**5.** Medicaments as claimed in any of claims 1 to 4, characterized in that they contain melphelan and 3-amino-1-hydroxypropane diphosphonic acid, preferably in the form of its disodium salt.

**6.** A process for the production of the medicament claimed in any of claims 1 to 5, characterized in that the cytostatic agents or therapeutic agents for the hormone therapy of tumours and 3-amino-1-hydroxypropane diphosphonic acid are mixed.

**7.** The use of a combination of cytostatic agents or hormone therapeutic agents and phosphono derivatives according to any of claims 1 to 6 for the production of a medicament for the chemotherapy of tumours.

## Revendications

1°) Médicament renfermant une combinaison d'un agent cytostatique ou d'agents médicamenteux pour la thérapie hormonale des tumeurs, et d'un dérivé phosphono caractérisé en ce qu'il s'agit pour le dérivé phosphono, des acides 3-amino-1-hydroxypropanedi phosphonique (APD) et en ce que l'agent cytostatique ou les médicaments hormonaux pour la thérapie hormonale des tumeurs, et l'acide 3-amino-1-hydroxypropanediphosphonique sont en rapports molaires de 25 : 1 à 1 : 20 et que les composés phosphono ainsi qu'éventuellement les agents cytostatiques ou les médicaments hormonaux se présentent sous forme de leurs sels compatibles pharmaceutiquement, le cas échéant ensemble avec des supports pharmaceutiques habituels et/ou des agents de dilution.

2°) Médicament selon la revendication 1, caractérisé en ce que l'acide 3-amino-1-hydroxypropanediphosphonique Se présente sous forme de leurs sels neutres, en particulier leurs sels alcalin et alcalino-terreux.

3°) Médicaments selon la revendication 1 ou 2, caractérisés en ce qu'ils renferment comme agent cytostatique, du Melphalan, du Chlorambucil, de la Dacarbacine, le Methotrexate, le Cyclophosphamide, la Vincristine, le Trophosphamide, l'Iphosphamide, le 5-fluorouracide, la lomustine, l'hydroxy Urée, le Cisplatine, le Carboplatine, l'hexaméthylène mélamine, la Daunorubicine, la Vinblastine et/ou la Zorubicine.

4°) Médicaments selon une des revendications 1 à 3, caractérisé en ce qu'ils renferment comme médicaments pour l'hormonothérapie des tumeurs du Tamoxifène, du Droloxifène et/ou de l'acétate de Méthroxyprogestérone.

5°) Médicaments selon une des revendications 1 à 4, caractérisés en ce qu'ils présentent un contenu en Melphalan et en Acide 3-amino-1-hydroxypropanediphosphonique, de préférence sous forme de leur sel disodique.

6°) Procédé d'obtention d'un médicament selon l'une des revendications 1 à 5, caractérisé en ce que l'on mélange les agents cytostatiques ou les médicaments pour l'hormonothérapie des tumeurs et l'acide 3-amino-1-hydroxypropanediphosphonique.

7°) Utilisation d'une combinaison d'agent cytostatique ou de médicaments hormonaux et de dérivés phosphono conformément à l'une des revendications 1 à 6 en vue de l'obtention d'un médicament pour la chimiothérapie des tumeurs.

Abb. 1 a

Zeit (Wochen) Kontrolle (100) = 3,85 Tumor

Abb. 1 b

Zeit (Wochen) ; ADP 11,75 mg/kg (100 % = 3.1)

EP 0 401 243 B1

8

Abb. 1 c

Zeit (Wochen) ; MEL 0,6 mg/kg (100 % = 2.8)

EP 0 401 243 B1

Abb. 1 d

Zeit (Wochen) ; APD + MEL 0,6 mg/kg (100 % = 3.8)

EP 0 401 243 B1

Abb. 1 e

Zeit (Wochen) ; APD + MEL 0,3 mg/kg (100 % = 3.3)

EP 0 401 243 B1

Abb. 2 a

APD

**Kontrolle**

O 23.5 mg/kg

□ 11.75 mg/kg

▽ 5.88 mg/kg

Mittleres Tumorvolumen (cm³)

Therapiedauer

Zeit (Wochen)

EP 0 401 243 B1

12

Abb. 2 b
MEL

Abb. 2 c
APD + MEL

Kontrolle
○ 11.75 mg/kg + 0.6 mg/kg
□ 11.75 mg/kg + 0.3 mg/kg
▽ 5.88 mg/kg + 0.3 mg/kg

Therapiedauer

Zeit (Woche.

Mittleres Tumorvolumen (cm³)